# EUROPEAN PATENT SPECIFICATION

(11) **EP 0 437 346 B1**
(45) Date of publication and mention of the grant of the patent: **24.04.1996**
(21) Application number: 91300142.6
(22) Date of filing: 09.01.1991
(51) Int. Cl.: C12P 1/02, A61K 35/84

(54) **Anti-viral fraction of aqueous Lentinus edodes extract**
Antivirale Fraktion von einem wässerigen Extrakt von Lentinus edodes
Fraction antivirale d'un extrait aqueux de Lentinus edodes

(30) Priority: 10.01.1990 JP 3818/90
(43) Date of publication of application: 17.07.1991
(73) Proprietor: NIHON CHEMICAL RESEARCH K. K., Hyogo 659 (JP); NODA SHOKUKIN KOGYO K. K., Chiba 278 (JP)
(72) Inventor: Koga, Junichi, Kobe, Hyogo 673 (JP); Ohashi, Yasuhiro, Noda, Chiba 278 (JP); Hiratani, Hajime, Sennan-gun, Osaka 599-02 (JP)
(74) Representative: Burford, Anthony Frederick

(56) References cited:
- EP-A- 0 154 066
- EP-A- 0 382 551
- GB-A- 2 023 131
- BIOCHEMICAL & BIOPHYSICAL RESEARCH COMMUNICATIONS, vol. 160, no. 1, 14 April 1989, Duluth, MN (US); S. HARUMI et al., pp. 367-373/
- JAPANESE JOURNAL OF BACTERIOLOGY, vol. 41, no. 3, 1986; M. TAKEHARA et al., p. 634;

## Description

The present invention relates to an inhibitor of the proliferation of herpes viruses in cells infected with them and of the reactivation of latent herpes viruses and the reinfection following it in a living body which has received a latent infection characteristic of these viruses.

The human herpes virus is a typical envelop virus having double-stranded DNA in its nucleocapsid, and is known to cause a variety of affections to an organism infected with it. In this category of human herpes viruses, these viruses are known: herpes simplex viruses type 1 and 2 (HSV1 and 2), varicella-herpes zoster viruses (VZV), cytomegaloviruses (CMV), Epstein-Barr viruses (EBV) and human herpes virus type 6 (HHV6). These viruses are characterized in that after a living body is infected with them first in its early childhood, they infect it latently, that is, continue to be alive as long as the living body lives. As the immunity of the organism changes, their affection recurs and is cured repeatedly throughout its life. In this course, some affections caused by the viruses are so grave that their infected living bodies die. Concerning immunosuppression during organ transplantation, the reinfection and reactivation of this virus affect the result of the operation of organ transplantation itself, so the development of an antiviral agent against herpes viruses is urgently needed for such operations. This virus is also known for causing a fatal affection or a sequela such as cerebritis to a fetus or newborn child by infection of mother and fetus by way of transplacental infection. More and more details are known of its relation with the manifestation of symptoms of venereal diseases such as genital herpes (herpes simplex virus type 2), epipharynx cancer, Burkitt lymphoma (EB virus), Kaposi sarcoma (CMV) and AIDS (HHV6). Recently in the course of development molecular biology has made a large number of discoveries related to herpes virus components. Information has been growing on the mechanism and role of glycoprotein specific to these viruses, enzyme activity in them such as thymidine kinase activity, the characteristic mechanism of genome DNA, latent infection, the base sequences related to the canceration of cells and so forth. However, many points remain to be explained in connection with herpesviruses' life cycle seen in the relation between this virus, its infected cell and the immunity of the organism during latent infection and reinfection, being obstacles to developing an inhibitor against the virus.

As understood from the above, this virus is a dangerous one which has very high pathogenicity, and thus a therapeutic agent against it is urgently needed. However, aciclovir is the only remedy now in use. To make matters worse, the application of the remedy is limited, since it has side effects such as teratogenic effect because it is an antagonist to nucleic acid synthesis.

Since olden times, various physiological active substances arising from mushrooms have been known, and some are used today as medicine. For example, Klestin (Yanagawa et al., Cancer and Chemotherapy 11, 2155, 1985), Lentinan (Suga et al., Cancer Research 44, 5162, 1984),and Shizofillan are used as antitumor agents. The active principles of these agents are polysaccharides or glycoprotein arising from bacteria, and their effect is thought to be by a biological response modifier (BRM) stimulating the immune system of a living body. These agents have no directly antiviral effect.

Recently, it has been reported that polysaccharides having sulfuric acid radicals, for instance dextran sulfuric acid, show antiviral activity against AIDS viruses (Mitsuya et al., Science 240, 646, 1988). Further, Tochikura and his group suggest that LEM, which is warm water extract of cultured Lentinus edodes mycelia (Med. Microbiol. Immunol. 177, 235, 1988), has the same kind of activity. Takehara et al (Japanese Journal of Bacteriology 41, 634, 1986) report that polysaccharides prepared by purification of fruit bodies of inter alia Lentinus edodes were tested for anti-herpes virus activity.

The method of producing LEM is already described in JP-A-53-10117. Various kinds of activity in vivo shown by LEM have been disclosed. This material has been in use as pesticide against infection with tobacco mosaic viruses. It has been reported to have clinical effect against hepatitis.

LEM is known to be made up of a mixture or complex of polysaccharides whose principal constituent is xylose and arabinose, water-soluble lignin and peptide and diverse inorganic substances, and has a long record of use as health food. With regard to origin as well as constitution, it is entirely different from Klestin, Lentinan and Shizofiran whose active constituents are conventional chemicals such as glucan and glycoprotein.

As described earlier, human herpes viruses cause a grave affection, but their remedies are too few and their application is limited. The development of a therapy for affections caused by these viruses and of therapeutic agent against them is, therefore, a very pressing problem.

Agents in current use against the viruses, aciclovir and BV-araU, which is at present being clinically experimented with, are analogues of nucleic acid, and target an enzyme existing in a nucleic acid synthesis system specific to the viruses in order to inhibit the nucleic acid synthesis. However, this does not necessarily work without affecting the nucleic acid synthesis system of the living body. They are really chemicals whose side effects cannot be denied. Thus, a safer and more effective medical agent is required today.

The object of the present invention is, therefore, to provide a safe and entirely new therapeutic agent different from conventional antiviral agents against herpes viruses, which is for inhibiting the proliferation of herpes viruses in cells and the reactivation of, and the reinfection with, these viruses in an organism which has received a latent infection with the viruses.

The inventors of the present invention discovered in LEM which is the starting material for the present active material or in its fractions the presence of fractions having absorption inhibiting activity against herpes simplex viruses, (see EP-A-0382551 published 16th August 1990 after the priority date claimed for the present Application). In the course of our further research, we found that one of the fractions obtained by purification processes starting with LEM, to our surprise, had activity for inhibition of the proliferation of these viruses in cells. The inventors also found that their reactivation was inhibited by intraperitoneal administration of this fraction (100 µg per day for 4 days) to a mouse which had received latent infection with them. In addition, their activation was inhibited by giving this fraction orally (500µg per day for 5 days) to a mouse similarly infected. This was utterly unexpected by the inventors. Considering the constituents of the fraction to be macromolecules (10,000 to 1,000,000 dalton) and its antigenicity, it seems difficult to devise any other medical application of this substance than oral administration and external use, so this experimental result was a very important discovery in terms of clinical application. An experiment on acute toxicity showed that this agent has a low toxicity: LD 50 = 15g per day (rat and mouse). The inventors of the active material named it JLS-18. It arises by fractionation from LEM which is warm water extract of cultured Lentinus edodes mycelia. The method of producing LEM is described in JP-A-53-10117. According to this method, Lentinus edodes mycelia are cultured in a medium made of bagasse and rice bran, and then warm water extraction of the active principle of the mycelia carried out just before the forming of fruit-bodies to obtain LEM. By fractionating this LEM as a starting liquid, JLS-18 is obtained. The fractionation is carried out by the combination of salting out, optional precipitation with organic solvent, ion exchange chromatography, hydrophobic chromatography and gel filtration chromatography. JLS-18, an active material thus obtained, has constant analytical values for carbohydrate composition, amino acid composition and lignin ratio, which suggests that these three constituents form a complex with their proportions constant. Since the molecular weight of this material ranges from several hundred to several million dalton according to the polymerization degrees of lignin forming the framework of the material, it is difficult to specify the molecular weight of the material. However, antiviral activity is strongest with the material weighing from ten thousand to hundred thousand dalton. Thus, material having such molecular weight is thought to achieve the objective of an antiviral agent against herpes viruses which is in accordance with the present invention.

The present invention is based upon the above-mentioned findings, and is embodied by an inhibitor of the proliferation of herpes viruses which contains JLS-18 and by an inhibitor, which also contains JLS-18, of the reactivation of, and the reinfection with,these viruses with which a living body has received a latent infection. These medical agents can be applied externally or by oral administration. For external use, it is preferable to have JLS-18 in pharmaceutical forms suitable for local administration to a region evidently, or possibly, infected with herpes viruses. Likely skin or membrane locations are on openings such as an oral cavity, pharynx, nasal cavity, eyelid, anus, rectum, urethra and vagina, wounds of the body or their periphery. An external use agent according to the present invention is for administration in pharmaceutical forms such as suppositories, troches, jelly, cream, cataplasms, ointment, plaster, liniment, solution, nebulae, aerosol and external use powder from which selections are to be made according to necessity. These external use medical agents are prepared by known methods. Since JLS-18 is very stable, its conservation is easy after it is dispensed as an external use agent.

JLS-18 is effective as an inhibitor of herpes virus reactivation and of reinfection following it when taken on the occasion of such reactivation or for the purpose of preventing it from occurring. Because of the high stability of JLS-18, there is no limitation to the selection for its pharmaceutical form from powder, granules, pills, tabellae, spirit, lemonades, etc.

JLS-18 is preferably used in such a way that an external use agent is constituted of about 0.01 to 1% of JLS-18. For oral administration, 10 mg to 10g a day is desirable.

The agent JLS-18 in accordance with the present invention not only inhibits herpes viruses from being bound to their target cells but inhibits their proliferation in cells, thus preventing the manifestation of diseases of their origin and the increase of infected regions in number and area. Further, the oral administration of this agent prevents the reactivation of herpes viruses with which an organism has received a persistent or latent infection, and also helps to alleviate symptoms after the reinfection with these viruses.

The following examples are further illustrative of the present invention.

### Example 1

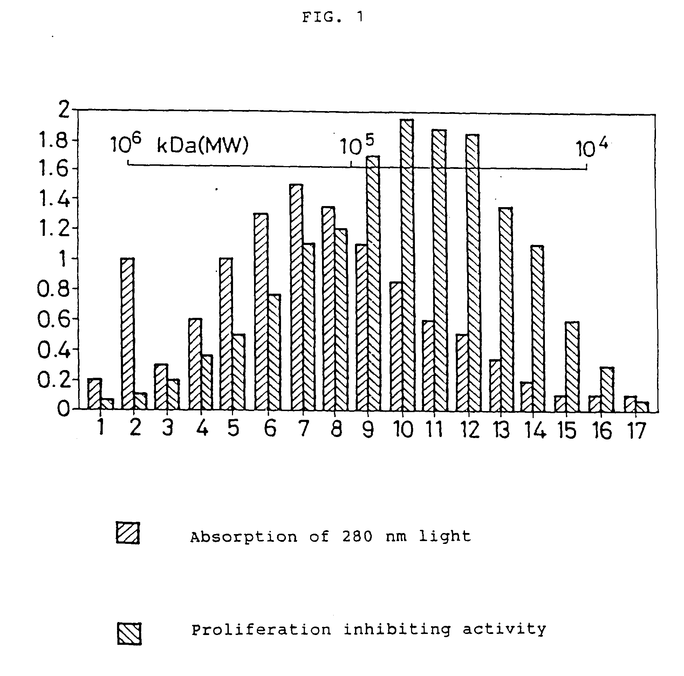

JLS-18 was derived from LEM by purification, first by adding 1L of 50mM phosphate buffer solution (pH 7.4) containing 2M ammonium sulfate to 1L of the LEM liquid, vigorously stirring the mixture and filtering it with a 0.45µm film. Then, the filtrate was passed through PhenylToyopearl (Tosoh) buffered by means of a 50mM phosphate buffer solution (pH 7.4) containing 1M ammonium sulfate. Absorbed antiviral activity constituents were fractionated while at the same time salt concentration was lowered gradually. As a result of this chromatography, the most hydrophobic fractions of the activity constituents were eluted within the range of phosphate concentration not more than 10mM. After concentrated by freeze-drying, these fractions were applied to molecular weight fractionation by gel filtration using HW-55 (Tosoh). Each fraction obtained was examined for antiviral activity (proliferation inhibiting activity) as described in Example 2, and we called a fraction (range) exhibiting the greatest activity as shown below by the name JLS-18. The result of the analysis of this agent is detailed in the table below, that of the chromatography in Figure 1 together with the result of the examination carried out in Example 2. Antiviral activity was distributed widely among the fractions obtained by the molecular weight fractionation, and this activity was greatest within the range of molecular weight 10,000 to 100,000 dalton. Then, the fractions having such molecular weight were desalted and freeze-dried.

Analytical values for JLS-18 were as follows:
(1) Constitution (%)

| | |
|---|---|
| Protein | 10 to 20 |
| Carbohydrate | 15 to 30 |
| Lignin | 65 to 75 |

(2) Amino acid constitution in the protein (%)

| | | | |
|---|---|---|---|
| Asx | 15.0 - 16.5 | Met | 0.3 - 0.7 |
| Thr | 6.5 - 7.2 | Ile | 3.8 - 4.9 |
| Ser | 6.5 - 7.3 | Leu | 4.9 - 6.6 |
| Glx | 15.0 - 17.5 | Tyr | 1.1 - 1.5 |
| Pro | 6.8 - 8.0 | Phe | 3.0 - 4.6 |
| Gly | 9.3 - 11.0 | Lys | 3.6 - 4.8 |
| Ala | 7.3 - 8.8 | His | 2.3 - 2.9 |
| Val | 5.9 - 6.5 | Arg | 0.7 - 1.1 |

(3) Carbohydrate constitution (%)

| | |
|---|---|
| Glucose | 35 to 55 |
| Xylose | 29 to 41 |
| Galactose | 2 to 7 |
| Arabinose | 12 to 20 |
| Mannose | 1 to 4 |

### Example 2

The manner in which virus proliferation inhibiting activity was assayed will be described here. First, the BSC-1 cell, which is a cell line derived from a monkey's kidney, was multiplied in the minimal essential medium (MEM) (Nissui Pharmaceutical Co.) containing 10% fetal calf serum (Flow Corp.) in a Petri dish 30mm in diameter (Corning Glass works). Fractions obtained by molecular fractionation as described in Example 1 were diluted ten times each in the MEM containing 10% fetal calf serum so as to be used as samples, and the cells in the Petri dish were cultured in medium containing each of the diluted fractions for 24 hours. A diluent of 100 Plaque forming unit (PFU) per milliliter of herpes simplex viruses was prepared by using a medium containing 2% fetal calf serum. After the cells were rinsed with Dulbecco's PBS, 0.5ml of the herpes simplex virus diluent was pipetted into each Petri dish and maintained at 37°C for two hours to absorb the viruses to the cells. After absorption, these cells were rinsed with PBS again, and 2ml of the MEM containing fresh 2% fetal calf serum was pipetted into each dish for 48 hours of culture. The cultured viruses were then processed by a 3000rpm centrifuge together with the cells for 10 minutes so that the supernatant fluid might be recovered from the content of each dish. The number of viruses in these supernatant fluids was evaluated using the plaque technique. This plaque technique was employed in the way described in Example 3.

Figure 1 is a graphical representation of the result of an examination, carried out in Example 2, of virus inhibiting activity exhibited by molecular weight fractions obtained in Example 1. This bar graph shows absorption of 280nm light by each of the fractions 1 to 17 and herpes virus proliferation inhibiting activity. Molecular weight estimated from the elution by gel filtration is shown in the upper part of the drawing.

### Example 3

Fractions obtained as described in Example 1 were applied to bioassay. When a mouse is first infected with herpes viruses in the eyelid, the mouse will soon have latent infection with them in the trigeminal ganglion. By removing such a ganglion from the mouse and then cultivating it in a test tube, it is possible to have the appearance of the viruses in the tissue so as to detect them. A bioassay examination was carried out by taking advantage of this nature of the virus, as will be described hereafter. 10,000,000 PFU of the F cell line of herpes simplex viruses 1 (multiplied in the vero cell) was pipetted into the eyes of six-week-old ICR mice. Beginning on the next day, 500µg per day of JLS-18 was administered orally for five days. The group of mice treated this way and a control group were made up of ten mice each. After they were fed for three weeks after the treatment, their trigeminal ganglia were removed, and each of the ganglia was cultivated for three days in a 199 medium (Flow Corp.) to which 5ml of 2% fetal calf serum had been added (for the reactivation of the latent viruses). After the cultivation, the tissues were rinsed with Dalbecco's phosphate-buffered physiological saline (Nissui Pharmaceutical Co.), and were minced in a 199 medium to which 2% fetal calf serum had been added, for the reactivated viruses to be extracted from the tissues. Then, these viruses were examined by applying the plaque assay system using vero cells derived from a monkey's kidney, as will be described from now on. Vero cells, which had been cultivated in an Eagle MEM (Nissui Pharmaceutical) to which 10% fetal calf serum had been added, were removed by using trypsin, and about 10,000 of them were placed in each hole in a 24 hole microplate, and then they were cultivated for two days so that they might adhere well to the plate. These cells were then applied for an hour to virus absorption using the virus extracts diluted stepwise to an appropriate concentration with an Eagle MEM to which 1% fetal calf serum had been added. After the cells were rinsed with Dalbecco's phosphate-buffered physiological saline, an Eagle MEM to which 1% fetal calf serum containing 0.2% agarose had been added was added to the cells in order to cultivate them for two days. The cultivated cells were inactivated with 10% formalin solution and stained with Methylene Blue, and the PFU quantity was counted under a microscope. The result was that no virus was detected in the group treated with JLS-18 though reactivation of viruses in trigeminal ganglia was found in all the members of the group (10 mice) not treated with it with the virus concentration not less than 1000PFU per ml. In addition to the above, changing the administration of the agent from oral to abdominal did not give the experiment any different result.

Details of the result is shown in the following table.

JLS-18′s activity for inhibiting latent infection virus reactivation is as follows:
(1) Oral administration

| Dose (µg per day) | Number of Mice | Positive as to Virus Infection (%) |
|---|---|---|
| 0 | 10 | 100 (10/10) |
| 100 | 10 | 30 ( 3/10) |
| 500 | 10 | 0 ( 0/10) |

(2) Abdominal administration

| Dose (µg per day) | Number of Mice | Positive as to Virus Infection (%) |
|---|---|---|
| 0 | 10 | 100 (10/10) |
| 30 | 10 | 60 ( 6/10) |
| 100 | 10 | 0 ( 0/10) |
| 500 | 10 | 0 ( 0/10) |

### Example 4

As described in Example 3, mice were infected with herpes simplex viruses and were fed four weeks without any treatment with medical agents so that they might receive latent infection. After their trigeminal ganglia were removed, the ganglia was cultivated for three days in the 199 medium to which 2% fetal calf serum had been added. 300µg per ml of JLS-18 had been added to the medium for cultivating a group to be treated. After the cultivation, reactivated viruses were recovered from the ganglion tissues, as in Example 2. From the group not treated, not less than 10,000 PFU per ml of viruses were recovered, but from the group treated with JLS-18, no virus was recovered.

### Example 5

JLS-18 preparations for oral administration were produced by dividing the agent into 500mg amounts and wrapping each amount in paper or a pouch.

### Example 6

100g of JLS-18 ointment was produced by dissolving 3g of JLS-18 in 10ml of physiological saline, adding hydrophilic ointment to it and then kneading it.

## Claims (Claims for the following Contracting State(s): CH, DE, FR, GB, IT, LI)

1. A substance which inhibits the proliferation of herpes viruses and the recurrence of affections caused by their latent infection, and is obtainable by fractionation by ion-exchange, hydrophobic and gel-filtration chromatography of a buffered pH 7.4 aqueous extract of cultured Lentinus edodes mycelia obtained by cultivating Lentinus edodes mycelia in a medium comprising bagasse and defatted rice bran, and extracting the resultant culture with warm water just before fruit-bodies are formed in the culture, said substance being a complex of 65 to 75% water-soluble lignin, 15 to 30% polysaccharide and 10 to 20% peptide and having a molecular weight of 10⁴ to 10⁶ dalton and an amino acid composition of
| | | | |
|---|---|---|---|
| Asx | 15.0 - 16.5 % | Met | 0.3 - 0.7 % |
| Thr | 6.5 - 7.2 % | Ile | 3.8 - 4.9 % |
| Ser | 6.5 - 7.3 % | Leu | 4.9 - 6.6 % |
| Glx | 15.0 - 17.5 % | Tyr | 1.1 - 1.5 % |
| Pro | 6.8 - 8.0 % | Phe | 3.0 - 4.6 % |
| Gly | 9.3 - 11.0 % | Lys | 3.6 - 4.8 % |
| Als | 7.3 - 8.8 % | His | 2.3 - 2.9 % |
| Val | 5.9 - 6.5 % | Arg | 0.7 - 1.1 %, |
and a sugar composition of
| | |
|---|---|
| Glucose | 35 - 55 % |
| Xylose | 29 - 41 % |
| Galactose | 2 - 7 % |
| Arabinose | 12 - 20 % |
| Mannose | 1 - 4 %. |

2. A pharmaceutical composition comprising a substance as claimed in Claim 1 and a pharmaceutically acceptable diluent or carrier.

3. A composition as claimed in Claim 2 for oral administration.

4. A composition as claimed in Claim 2 for external administration.

5. A composition as claimed in Claim 4 for topical adminstration.

6. A composition as claimed in Claim 4 in the form of a suppository.

7. A substance as claimed in Claim 1 for use as an active pharmaceutical substance.

8. The use of a substance as claimed in Claim 1 in the manufacture of a medicament for the treatment of herpes virus infections.

9. A use as claimed in Claim 8, wherein the infection is latent infection with herpes simplex viruses type 1 or 2 (HSV1 or 2), varicella-zoster viruses (VZV), or cytomegaloviruses (CMV).

## Claims (Claims for the following Contracting State(s): ES)

1. A method of obtaining a substance which inhibits the proliferation of herpes viruses and the recurrence of affections caused by their latent infection, said method comprising fractionating by ion-exchange, hydrophobic and gel-filtration chromatography a buffered pH 7.4 aqueous extract obtained by culturing Lentinus edodes mycelia in a medium comprising bagasse and defatted rice bran, and extracting the resultant culture with warm water just before fruit-bodies are formed in the culture to obtain a complex of 65 to 75% water-soluble lignin, 15 to 30% polysaccharide and 10 to 20% peptide and having a molecular weight of 10⁴ to 10⁶ dalton and an amino acid composition of
| | | | |
|---|---|---|---|
| Asx | 15,0 - 16.5 % | Met | 0.3 - 0.7 % |
| Thr | 6.5 - 7.2 % | Ile | 3.8 - 4.9 % |
| Ser | 6.5 - 7.3 % | Leu | 4.9 - 6.6 % |
| Glx | 15.0 - 17.5 % | Tyr | 1.1 - 1.5 % |
| Pro | 6.8 - 8.0 % | Phe | 3.0 - 4.6 % |
| Gly | 9.3 - 11.0 % | Lys | 3.6 - 4.8 % |
| Als | 7.3 - 8.8 % | His | 2.3 - 2.9 % |
| Val | 5.9 - 6.5 % | Arg | 0.7 - 1.1 %, |
and a sugar composition of
| | |
|---|---|
| Glucose | 35 - 55 % |
| Xylose | 29 - 41 % |
| Galactose | 2 - 7 % |
| Arabinose | 12 - 20 % |
| Mannose | 1 - 4 %. |

## Patentansprüche (Patentansprüche für folgende(n) Vertragsstaat(en): CH, DE, FR, GB, IT, LI)

1. Substanz mit Inhibitorwirkung gegen die starke Vermehrung von Herpesviren und Rückfallerkrankungen, die durch latente Infektionen der Herpesviren hervorgerufen werden, erhältlich durch die Fraktionierung durch Ionenaustausch, hydrophobe und Gelfitrationschromatographie eines auf pH 7,4 gepufferten, wäßrigen Extrakts aus kultivierten Lentinus edodes-Mycelien, der dadurch erhalten worden ist, daß die Mycelien von Lentinus edodes in einem Bagasse (zuckerfreie Zuckerrohrrückstände) und entfettete Reiskleie enthaltendem Medium kultiviert wurden, sowie durch Extraktion der so erhaltenen Kultur mit warmem Wasser unmittelbar vor der Ausbildung der Fruchtkörper in der Kultur, wobei die Substanz einen Komplex aus 65 bis 75 % wasserlöslichem Lignin, 15 bis 30 % Polysaccharid und 10 bis 20 % Peptid darstellt, deren Molekulargewicht 10⁴ bis 10⁶ Dalton beträgt, deren Aminosäurezusammensetzung
| | | | |
|---|---|---|---|
| Asx | 15,0 - 16,5 % | Met | 0,3 - 0,7 % |
| Thr | 6,5 - 7,2 % | Ile | 3,8 - 4,9 % |
| Ser | 6,5 - 7,3 % | Leu | 4,9 - 6,6 % |
| Glx | 15,0 - 17,5 % | Tyr | 1,1 - 1,5 % |
| Pro | 6,8 - 8,0 % | Phe | 3,0 - 4,6 % |
| Gly | 9,3 - 11,0 % | Lys | 3,6 - 4,8 % |
| Als | 7,3 - 8,8 % | His | 2,3 - 2,9 % |
| Val | 5,9 - 6,5 % | Arg | 0,7 - 1,1 % |
und deren Zuckerzusammensetzung
| | |
|---|---|
| Glucose | 35 - 55 % |
| Xylose | 29 - 41 % |
| Galaktose | 2 - 7 % |
| Arabinose | 12 - 20 % |
| Mannose | 1 - 4 % |
betragen.

2. Arzneimittel mit einem Gehalt an einer Substanz nach Anspruch 1 neben einem pharmazeutisch verträglichen Verdünnungsmittel oder Trägerstoff.

3. Mittel nach Anspruch 2 zur peroralen Verabreichung.

4. Mittel nach Anspruch 2 zur äußerlichen Verabreichung.

5. Mittel nach Anspruch 4 zur topischen Applikation.

6. Mittel nach Anspruch 4 in Form eines Suppositoriums.

7. Substanz nach Anspruch 1 zur Verwendung als wirksamer Arzneistoff.

8. Verwendung einer Substanz nach Anspruch 1 bei der Herstellung eines Arzneimittels zur Behandlung von Herpesvireninfektionen.

9. Verwendung nach Anspruch 8, bei der die Infektion einen latenten Infekt durch Herpes simplex-Viren vom Typ 1 oder 2 (HSV1 oder HSV2), Varicella zoster-Viren (VZV) oder Cytomegaloviren (CMV) darstellt.

## Patentansprüche (Patentansprüche für folgende(n) Vertragsstaat(en): ES)

1. Verfahren zum Erhalt einer Substanz mit inhibierender Wirkung gegen die starke Vermehrung von Herpesviren und Rückfallinfekte, die durch latente Infektionen der Herpesviren hervorgerufen werden, wobei das Verfahren die Schritte der Fraktionierung durch Ionenaustausch, hydrophobe und Gelfitrationschromatographie eines auf pH 7,4 gepufferten, wäßrigen Extrakts aus kultiviertem Mycel von Lentinus edodes umfaßt, der dadurch erhalten worden ist, daß Mycelien von Lentinus edodes in einem Bagasse (zuckerfreie Zuckerrohrrückstände) und entfettete Reiskleie enthaltendem Medium kultiviert wurden, sowie die Extraktion der so erhaltenen Kultur mit warmem Wasser unmittelbar vor der Ausbildung der Fruchtkörper in der Kultur umfaßt, wobei die Substanz einen Komplex aus 65 bis 75 % wasserlöslichem Lignin, 15 bis 30 % Polysaccharid und 10 bis 20 % Peptid darstellt, deren Molekulargewicht 10⁴ bis 10⁶ Dalton beträgt, deren Aminosäuren sich wie folgt:
| | | | |
|---|---|---|---|
| Asx | 15,0 - 16,5 % | Met | 0,3 - 0,7 % |
| Thr | 6,5 - 7,2 % | Ile | 3,8 - 4,9 % |
| Ser | 6,5 - 7,3 % | Leu | 4,9 - 6,6 % |
| Glx | 15,0 - 17,5 % | Tyr | 1,1 - 1,5 % |
| Pro | 6,8 - 8,0 % | Phe | 3,0 - 4,6 % |
| Gly | 9,3 - 11,0 % | Lys | 3,6 - 4,8 % |
| Als | 7,3 - 8,8 % | His | 2,3 - 2,9 % |
| Val | 5,9 - 6,5 % | Arg | 0,7 - 1,1 % |
und deren Zucker sich aus
| | |
|---|---|
| Glucose | 35 - 55 % |
| Xylose | 29 - 41 % |
| Galaktose | 2 - 7 % |
| Arabinose | 12 - 20 % |
| Mannose | 1 - 4 % |
zusammensetzen.

## Revendications (Revendications pour l'(les) Etat(s) contractant(s) suivant(s): CH, DE, FR, GB, IT, LI)

1. Substance qui inhibe la prolifération de virus de l'herpès et la récidive d'affections causées par leur infection latente, et qui peut être obtenue par fractionnement par chromatographie d'échange ionique, hydrophobe et de filtration sur gel d'un extrait aqueux, tamponné à un pH de 7,4, de mycéliums de *Lentinus edodes* cultivés, obtenu par culture de mycéliums de *Lentinus edodes* dans un milieu comprenant de la bagasse et du son de riz dégraissé et par extraction de la culture résultante avec de l'eau chaude juste avant que des corps-fructifères ne soient formé dans la culture, ladite substance étant un complexe de 65 à 75% de lignine hydrosoluble, de 15 à 30°A de polysaccharide et de 10 à 20% de peptide et ayant un poids moléculaire de 10⁴ à 10⁶ daltons et une composition en acides aminés de
| | | | |
|---|---|---|---|
| Asx | 15,0 - 16,5% | Met | 0,3 - 0,7% |
| Thr | 6,5 - 7,2% | Ile | 3,8 - 4,9% |
| Ser | 6,5 - 7,3% | Leu | 4,9 - 6,6% |
| Glx | 15,0 - 17,5% | Tyr | 1,1 - 1,5% |
| Pro | 6,8 - 8,0% | Phe | 3,0 - 4,6% |
| Gly | 9,3 - 11,0% | Lys | 3,6 - 4,8% |
| Als | 7,3 - 8,8% | His | 2,3 - 2,9% |
| Val | 5,9 - 6,5% | Arg | 0,7 - 1,1%, |
et une composition en sucres de
| | |
|---|---|
| Glucose | 35 - 55% |
| Xylose | 29 - 41% |
| Galactose | 2 - 7% |
| Arabinose | 12 - 20% |
| Mannose | 1 - 4%. |

2. Composition pharmaceutique, comprenant une substance selon la revendication 1 et un diluant ou un support acceptable pharmaceutiquement.

3. Composition selon la revendication 2 pour administration orale.

4. Composition selon la revendication 2 pour administration externe.

5. Composition selon la revendication 4 pour administration topique.

6. Composition selon la revendication 4 sous la forme d'un suppositoire.

7. Substance selon la revendication 1 à usage comme substance pharmaceutique active.

8. Utilisation d'une substance selon la revendication 1 dans la préparation d'un médicament pour le traitement d'infections par un virus de l'herpès.

9. Utilisation selon la revendication 8, dans laquelle l'infection est une infection latente par des virus d'herpès simplex type 1 ou 2 (HSV1 ou 2), des virus de la varicelle-zona (VZV) ou des cytomégalovirus (CMV).

## Revendications (Revendications pour l'(les) Etat(s) contractant(s) suivant(s): ES)

1. Procédé pour l'obtention d'une substance qui inhibe la prolifération de virus de l'herpès et la récidive d'affections causées par leur infection latente, ledit procédé comprenant le fractionnement par chromatographie d'échange ionique, hydrophobe et de filtration sur gel d'un extrait aqueux, tamponné à un pH de 7,4, obtenu par culture de mycéliums de *Lentinus edodes* dans un milieu comprenant de la bagasse et du son de riz dégraissé et par extraction de la culture résultante avec de l'eau chaude juste avant que des corps-fructifères ne soient formés dans la culture, pour obtenir un complexe de 65 à 75% de lignine hydrosoluble, de 15 à 30% de polysaccharide et de 10 à 20% de peptide, ayant un poids moléculaire de 10⁴ à 10⁶ daltons et une composition en acides aminés de
| | | | |
|---|---|---|---|
| Asx | 15,0 - 16,5% | Met | 0,3 - 0,7% |
| Thr | 6,5 - 7,2% | Ile | 3,8 - 4,9% |
| Ser | 6,5 - 7,3% | Leu | 4,9 - 6,6% |
| Glx | 15,0 - 17,5% | Tyr | 1,1 - 1,5% |
| Pro | 6,8 - 8,0% | Phe | 3,0 - 4,6% |
| Gly | 9,3 - 11,0% | Lys | 3,6 - 4,8% |
| Als | 7,3 - 8,8% | His | 2,3 - 2,9% |
| Val | 5,9 - 6,5% | Arg | 0,7 - 1,1%, |
et une composition en sucres de
| | |
|---|---|
| Glucose | 35 - 55% |
| Xylose | 29 - 41% |
| Galactose | 2 - 7% |
| Arabinose | 12 - 20% |
| Mannose | 1 - 4%. |
